**Europäisches Patentamt**

**European Patent Office**

**Office européen des brevets**

(19)

(11) Veröffentlichungsnummer: **0 064 758**
**B1**

(12) **EUROPÄISCHE PATENTSCHRIFT**

(45) Veröffentlichungstag der Patentschrift:
08.08.84

(51) Int. Cl.³: **G 01 N 33/72**

(21) Anmeldenummer: **82104046.6**

(22) Anmeldetag: **10.05.82**

(54) Verfahren zur Bestimmung des Gehalts an glykosiliertem Hämoglobin bei der Langzeitkontrolle des Blutzuckerspiegels.

(30) Priorität: **13.05.81 DE 3119046**

(43) Veröffentlichungstag der Anmeldung:
**17.11.82 Patentblatt 82/46**

(45) Bekanntmachung des Hinweises auf die Patenterteilung:
**08.08.84 Patentblatt 84/32**

(84) Benannte Vertragsstaaten:
**AT BE CH FR GB IT LI NL SE**

(56) Entgegenhaltungen:
**US - A - 4 243 534**

**CHEMICAL ABSTRACTS, Band 96, nr. 19, 10. Mai 1982, Seite 401, Nr. 158761r, Columbus, Ohio, USA D.M. NATHAN et al.: "Rapid method for eliminating labile glycosylated hemoglobin from the assay for hemoglobin A"**
**CHEMICAL ABSTRACTS, Band 94, Nr. 11, 11. März 1981, Seite 351, Nr. 79890n, Columbus, Ohio, USA**
**CHEMICAL ABSTRACTS, Band 96, Nr. 19, 10. Mai 1982, Seite 369, Nr. 158417h, Columbus, Ohio, USA M. TROVATI et al.: "Rapid changes of glycosylated hemoglobin in vitro and in vivo"**

(73) Patentinhaber: **PANCHEM Gesellschaft für chemische Produkte mbH, Schlossstrasse 3, D-8751 Kleinwallstadt (DE)**

(72) Erfinder: **Niederau, Cornelia, Werderstrasse 17, D-5600 Wuppertal 11 (DE)**
Erfinder: **Reinauer, Hans, Brinckmannstrasse 37, D-4000 Düsseldorf (DE)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

## Beschreibung

Die Erfindung betrifft ein Verfahren und ein Reagenz zur Bestimmung des Gehalts an glykosiliertem Hämoglobin bei der Langzeitkontrolle des Blutzuckerspiegels durch Hämolyse der Erythrozyten, chromatographische Trennung der Hämoglobinfraktionen und deren kolorimetrische Messung.

Die Tatsache, daß bei diabetischen Patienten eine Erhöhung des Glykohämoglobingehalts in den roten Blutkörperchen eintritt, ermöglicht eine Bestimmungsmethode, um die Stoffwechsellage von Diabetikern zu kontrollieren. Es bilden sich nämlich durch eine nichtenzymatische, irreversible Reaktion der Glucose im Blut mit einer der endständigen Aminogruppen der Proteinketten sowie mit $\varepsilon$-Aminogruppen der Lysine des Hämoglobins in den Erythrozyten glykosilierte Hämoglobinfraktionen, die sich chromatographisch von normalem Hämoglobin abtrennen lassen. Die Glykoproteinsynthese findet während der gesamten Erythrozytenhalblebensdauer von 120 Tagen statt, wobei diese Reaktion von der durchschnittlichen Glucosekonzentration und ihrer Dauer im Blut abhängig ist. Die Glykohämoglobin-Gesamtfraktion $HbA_1$ besteht aus drei Unterfraktionen $HbA_{1a} + HbA_{1b} + HbA_{1c}$. Das als $HbA_{1c}$ bezeichnete glykosilierte Hämoglobin eignet sich besonders gut zu dieser Kontrolle des Blutzuckers. Daher ist der Gehalt an $HbA_{1c}$, das in einer Menge von 3—6% im Gesamthämoglobin normaler Erwachsener vorhanden ist, ein Indiz für die Höhe des durchschnittlichen Blutzuckerspiegels während der Lebensdauer der Erythrozyten. Die Bestimmung des $HbA_{1c}$-Wertes erfolgt nach Hämolyse der Erythrozyten u. a. mittels einer chromatographischen Trennung der Hämoglobinfraktionen an Makrosäulen (Trivelli et al, New Engl. J. Med. 284, 353—357 (1971) oder an Mikrosäulen und anschließenden kolorimetrischen Messungen im Spektrophotometer bei ca. 415 nm.

Die Trennung über Mikrosäulen ersetzte die in Praxi zu aufwendige Makrosäulentechnik weitgehend, nachdem sich herausstellte, daß die Gesamtfraktion der glykosilierten Hämoglobine $HbA_{1a+1b+1c}$ ebenfalls den Langzeitblutzuckerwert in guter Korrelation wiedergibt und dadurch die Aufspaltung in die Unterfraktionen, die auf den Mikrosäulen nicht möglich ist, für diagnostische Zwecke unterbleiben kann (vergl. Koenig, R. J., C. M. Peterson, R. L. Jones, C. Saudek, M. Lehrman, und A. Cerami: Correlation of glucose regulation and hemoglobin $A_{1c}$ in diabetes mellitus, in N. Eng. J. Med. 295, 417—420 [1976]; Fitzgibbons, J. F., R. D. Koler und R. T. Jones: Red cell agerelated changes of hemoglobins $A_{1a+b}$ and $A_{1c}$ in normal and diabetic subjects, in J. Clin. Inves. 58, 820—824 [1976]). Alle kommerziell von verschiedenen Herstellern bereitgestellten Mikrosäulenbestimmungssets bedienen sich derselben grundsätzlichen Technik. Die $HbA_1$-Gesamtfraktion wird über einen Ionenaustauscher mit Hilfe eines Puffers, dessen pK-Wert auf die Ionenstärke des Austauschers abgestimmt ist, von dem Resthämoglobin abgetrennt. Als Bezugsgröße wird dann anschließend mit einer zweiten Elutionslösung das Resthämoglobin von der Säule gewaschen oder aus einem entsprechenden Hämolysataliquot das Gesamthämoglobin bestimmt. Aus beiden Werten wird sodann der relative Prozentsatz des $HbA_1$ am Gesamthämoglobin errechnet. Im Prinzip liegt dieselbe Technik auch Bestimmungsmethoden zugrunde, die das an das Austauschharz gebundene Hämoglobin abzentrifugieren (z. B. Leeco Diagnostics).

Bekannt ist, daß die Glykosilierung des Hämoglobins in den Erythrozyten über einen zweistufigen Prozeß abläuft, wobei u. a. zunächst die Aldehydgruppe der Glucose mit der Aminogruppe des endständigen Valins der $\beta$-Kette des Hämoglobins zur Aldiminform (Schiffsche Base) reagiert. Dieses relativ instabile Aldiminderivat lagert sich dann aufgrund einer Amadori-Umlagerung in die relativ stabile Ketoaminform um. Kurzfristige Blutzuckerschwankungen während des Tages, an dem der Test vorgenommen wird, können daher zu einer entsprechend hohen Bildungsrate des instabilen Aldimins führen, wodurch die Beurteilung des $HbA_1$-Wertes als Langzeitparameter der diabetischen Stoffwechselkontrolle in Frage gestellt würde (vergl. Schernthaner, Dtsch. Med. Wschr. 106, 259—261 [1981]; J. Ditzel, Diabetologia 19, 403—404 [1980]).

Aufgabe der Erfindung ist es, diese $HbA_1$-Bestimmungsmethoden zu verbessern, indem man diese labile Aldiminform vor der Auftrennung der Hämoglobinfraktionen eliminiert.

Diese Aufgabe wird durch das Verfahren gemäß Patentanspruch 1 gelöst. Die Erfindung betrifft auch ein Reagenz wie es in Anspruch 6 definiert ist.

Es wurde überraschenderweise festgestellt, daß es in einfacher Weise gelingt, durch ein konkurrierendes Angebot an einem primären Amin in Gegenwart einer Hydrazinverbindung die labile Aldiminform der $HbA_1$-Fraktion unter Bildung der entsprechenden Schiffschen Basenverbindung durch »Transschiffisierung« zu spalten, die aber die Bestimmung der stabilen Glucose-Ketoaminform des Hämoglobins bei der chromatographischen Trennung bzw. spektroskopischen Messung nicht mehr stört. Als primäre Amine eignen sich erfindungsgemäß alle gut wasserlöslichen Aminverbindungen mit mindestens einer freien $NH_2$-Gruppe, z. B. Hydroxylamin, Alkylamine, wie Äthyl- oder Propylamin, Hydroxyalkylamine, wie Äthanolamin oder Aminosäuren. Als solche sind geeignet das Lysin und das Valin. Aber auch Pyridoxamin oder Anilin sind hierfür brauchbar. Als Hydrazinverbindungen eignen sich das Hydrazin selbst sowie seine substituierten Verbindungen, wie Phenylhydrazin, Semicarbazid.

Vorzugsweise erfolgt die Zugabe der Kombination von primärem Amin und Hydrazinverbindung bei der notwendigen Hämolysestufe der Erythrozyten zur Freilegung des Hämoglobins indem man ein Hämolysierungsreagens verwendet, welches sowohl die primäre Aminoverbindung als auch die Hydrazinverbindung enthält.

# 0 064 758

Beispiel

Ein besonders geeignetes Reagens enthält folgende Komponenten:

| | |
|---|---|
| 200 mM | Semicarbazid-hydrochlorid |
| 20 mM | Lysin |
| 20 mM | Valin |
| 50 mM | Phosphatpuffer pH = 6,6 sowie |
| 0,813 mM | Digitonin je Liter der wäßrigen Reagenslösung. |

Zur Durchführung der erfindungsgemäßen Bestimmung des $HbA_1$-Wertes soll im allgemeinen frisch entnommenes Blut verwendet werden. Wenn dies nicht möglich ist, soll man dem Blut als Antikoagulans möglichst Heparin oder Äthylendiamin-tetraessigsäure zusetzen, wodurch die Bestimmung nicht gestört wird. Das so stabilisierte Blut kann maximal eine Woche im Eisschrank gelagert werden. Dem Blut, das etwa Zimmertemperatur (20—23° C) aufweisen soll, wird dann mit dem erfindungsgemäß das primäre Amin und die Hydrazinverbindung enthaltenden Hämolysereagens versetzt, worauf kurz gemischt wird. Nach ca. 10 Minuten langem Stehen bei Zimmertemperatur, wobei die Hämolyse und die erfindungsgemäße Spaltung der labilen Aldiminverbindung eintritt, wird in an sich bekannter Weise das hämolysierte Blut auf die übliche Trennsäule gegeben und dadurch die Hämoglobinfraktionen von einander getrennt. Die schnell-laufende Fraktion ($HbA_1$) wird dann photometrisch bestimmt.

Bei Ersatz des von den Herstellern angegebenen Hämolysierungsreagens durch das erfindungsgemäße Reagens wird im wesentlichen nach der Arbeitsanleitung des betreffenden Herstellers verfahren. Bei Blutzuckerwerten von unter 500 mg/dl wird das erfindungsgemäße Reagens mit einem gleichen Volumen Aqua dest. verdünnt und in gleicher Menge, wie vom Hersteller angegeben, dem ebenfalls beibehaltenen Blutprobenvolumen beigegeben. Bei Blutzuckerwerten von über 500 mg/dl wird zunächst die Hälfte des benötigten Hämolysierungsreagensvolumens durch die konzentrierte Form des erfindungsgemäßen Reagens ersetzt und nach ca. 10 Minuten ein entsprechendes Volumen Aqua dest. hinzugefügt.

Der Gehalt an glykosiliertem Hämoglobin wird wie in der systemspezifischen Arbeitsanleitung ermittelt. Durch die erfindungsgemäße Ausschaltung des labilen Aldimin-Zwischenprodukts der Glykosilierungsreaktion des Hämoglobins in den Erythrozyten gibt der gemessene Wert ausschließlich den Gehalt an der stabilen Ketoaminform des glykosilierten Hämoglobins wieder, die dem von kurzzeitigen hyperglykämischen Schwankungen befreiten Langzeitwert des Blutzuckerspiegels entspricht. Hierdurch läßt sich nun eine wesentlich bessere und sicherere Kontrolle und Einstellung der Diabetes-Patienten durchführen, als dies bisher mit der chromatographisch-photometrischen Kontrolle des glykosilierten Hämoglobins der Fall war. Auch postprandiale Analysen sind störungsfrei möglich.

Folgender Versuch zeigt die Wirksamkeit der Beseitigung der labilen Aldiminform durch das erfindungsgemäße Reagens.

Es wurde im Nüchternblut von 4 verschiedenen Diabetes-Patienten der Gehalt an glykosiliertem Hämoglobin bestimmt. Entsprechende Blutproben wurden 2 Stunden lang mit zusätzlicher Glucose in einer Menge von 50 mM bei 37° C inkubiert und dann die Messung wiederholt, wobei die Untersuchungen mit a) normalem Hämolysereagens (0,813 mM Digitonin/Liter) — Phosphatpuffer pH = 6,6) und b) dem erfindungsgemäßen Reagens durchgeführt wurden, wie es im Beispiel angegeben ist.

Die nachfolgende Tabelle zeigt den ermittelten Gehalt an glykosiliertem Hämoglobin, bezogen auf das Gesamthämoglobin.

Tabelle

| | | | | |
|---|---|---|---|---|
| Ausgangswerte des Glocose-Hämoglobins | 6,73% | 9,5% | 11,1% | 11,86% |
| a) Glucose-Hämoglobin nach 2 Stunden Glykosilierung mit üblichem Hämolysereagens | 8,75% | 11,9% | 14,3% | 15,16% |
| b) Glucose-Hämoglobin nach 2 Stunden Glykosilierung mit erfindungsgemäßem Reagens | 7,66% | 10,5% | 11,16% | 11,81% |

Es ist ersichtlich, daß die nach künstlicher Hyperglykämie gemessenen hohen Werte an Glucose-Hämoglobin wieder annähernd auf den Ausgangswert zurückgeführt wurden, da die dabei zunächst intermediär gebildete instabile Aldiminform mit dem erfindungsgemäßen Reagens eliminiert wurde. Die dennoch, insbesondere bei niedrigen Ausgangswerten, gemessenen erhöhten Glucose-Hämoglobinwerte zeigen, daß bei der Glykosilierung ein Teil der instabilen Aldiminform sich bereits in die stabile Ketoaminform umgelagert hatte.

3

# 0 064 758

## Patentansprüche

1. Verfahren zur Bestimmung des Gehalts an glykosiliertem Hämoglobin bei der Langzeitkontrolle des Blutzuckerspiegels durch Hämolyse der Erythrozyten, chromatographische Trennung der Hämoglobinfraktionen und deren kolorimetrische Messung, dadurch gekennzeichnet, daß zur Ausschaltung der instabilen Glucose-Aldimin-Hämoglobinverbindungen das Hämolysat mit einer wasserlöslichen primären Aminverbindung und einer Hydrazinverbindung umgesetzt wird, worauf das so vorbehandelte Hämolysat auf die Chromatographiersäule aufgegeben wird.

2. Verfahren nach Anspruch 1, dadurch gekennzeichnet, daß die Hämolyse und die Umsetzung der primären Amine und der Hydrazinverbindung gleichzeitig durchgeführt werden.

3. Verfahren nach Anspruch 1 oder 2, dadurch gekennzeichnet, daß als Aminverbindung Hydroxylamin, Alkylamine, Anilin, Pyridoxamin oder Aminosäuren verwendet werden.

4. Verfahren nach Anspruch 3, dadurch gekennzeichnet, daß als Aminosäure Lysin und/oder Valin verwendet wird.

5. Verfahren nach Anspruch 1—4, dadurch gekennzeichnet, daß als Hydrazinverbindung Hydrazin, Phenylhydrazin oder Semicarbazid verwendet wird.

6. Reagens zur Durchführung des Verfahrens nach Anspruch 2—5, dadurch gekennzeichnet, daß es Lysin, Valin, Semicarbazid, Digitonin sowie Phosphatpuffer zur Einstellung auf einen pH-Wert von ca. 6,6 enthält.

7. Reagens nach Anspruch 6, dadurch gekennzeichnet, daß es 20 mM Lysin, 20 mM Valin, 200 mM Semicarbazidhydrochlorid, 50 mM Natriumphosphatpuffer (für pH = 6,6) sowie 0,813 mM Digitonin je Liter der wäßrigen Lösung enthält.

## Claims

1. A process for detecting the content of glycosylated haemoglobin in a long-term control of the blood-sugar level by haemolysis of the erythrocytes, chromatographically separating the haemoglobin fractions and colorimetrically measuring the same characterized in that for eliminating the instable glucose-aldimine-haemoglobin compounds, the haemolysate is reacted with a water-soluble, primary amine compound and a hydrazine compound, the thus pre-treated haemolysate being then applied onto the cromatographic solumn.

2. The process as defined in claim 1 characterized in that the haemolysis and the reaction of the primary amines and of the hydrazine compound are carried out simultaneously.

3. The process as defined in claim 1 or claim 2 characterized in that as amine compound hydroxyl amine, alkyl amines, aniline, pyridoxamine or amino acids are used.

4. The process as defined in claim 3 characterized in that the amino acid used is lysine and/or valine.

5. The process as defined in claims 1 to 4 characterized in that as hydrazine compound hydrazine, phenyl-hydrazine or semi-carbizide is used.

6. A reagent for carrying out the process as defined in claims 1 to 5 characterized in that it contains lysine, valine, semi-carbizide, digitonin, as well as phosphate buffers for the adjustment to a pH of about 6.6.

7. The reagent as defined in claim 6 characterized in that it contains 20 mM lysine, 20 mM valine, 200 mM semicarbizide hydrochloride, 50 mM sodium phosphate buffer (for pH = 6.6) as well as 0.813 mM digitonin per one liter of the aqueous solution.

## Revendications

1. Procédé pour déterminer la teneur en hémoglobine glucosylée dans le contrôle à long terme de la teneur en sucre du sang par hémolyse des érythrocytes, séparation chromatographique des fractions d'hémoglobine et dosage colorimétrique de ces fractions, caractérisé en ce que, pour exclure les composés instables du type glucose-aldimine-hémoglobine, on fait réagir l'hémolysat avec une amine primaire hydrosoluble et une hydrazine, après quoi on fait passer l'hémolysat traité sur la colonne chromatographique.

2. Procédé selon la revendication 1, caractérisé en ce que l'hémolyse et la réaction avec l'amine primaire et l'hydrazine sont effectuées simultanément.

3. Procédé selon la revendication 1 ou 2, caractérisé en ce que l'on utilise en tant qu'amine l'hydroxylamine, une alkylamine, l'aniline, la pyridoxamine ou un amino-acide.

4. Procédé selon la revendication 3, caractérisé en ce que l'on utilise en tant qu'amino-acide la lysine et/ou la valine.

5. Procédé selon les revendications 1 à 4, caractérisé en ce que l'on utilise en tant qu'hydrazine, l'hydrazine non substituée, la phénylhydrazine ou le semi-carbazine.

6. Réactif pour la mise en oeuvre du procédé selon les revendications 2 à 5, caractérisé en ce qu'il contient de la lysine, de la valine, du semi carbazide, de la digitonine et un tampon phosphaté pour

4

réglage d'un pH d'environ 6,6.

7. Réactif selon la revendication 6, caractérisé en ce qu'il contient 20 millimoles de lysine, 20 millimoles de valine, 200 millimoles de chlorhydrate de semicarbazide, 50 millimoles de tampon au phosphate de sodium (pour pH 6,6) et 0,813 millimoles de digitonine par litre de la solution aqueuse.